# EUROPEAN PATENT APPLICATION

(11) **EP 4 241 787 A2**
(43) Date of publication of application: **13.09.2023**
(21) Application number: 23182605.8
(22) Date of filing: 03.03.2020
(51) Int. Cl.: A61K 41/00

(54) **METHOD AND DEVICE FOR GUT MICROBIOTA MANIPULATION**

(30) Priority: 04.03.2019 US 201962813543 P
(62) Divisional of application: 20766447.5
(71) Applicant: Photopill Medical Ltd., 7660660 Rehovot (IL)
(72) Inventor: BEN YEHUDA, Sharon, 7660660 Rehovot (IL); BEN YEHUDA, Ram, 7660660 Rehovot (IL)
(74) Representative: Lecomte & Partners

(57) **Abstract**

The present disclosure generally provides a system for manipulating human gut microbiota *in-situ,* comprising a swallowable light emitting vehicle configured to move through the GI tract of a human subject; a software; a software operated controller and a plurality of protocols for manipulating the gut microbiota, wherein each protocol comprises a predefined set of conditions, and, wherein said set of conditions comprises at least one illumination episode at the wavelength in the range of 400nm to 900nm.

## Description

### TECHNICAL FIELD OF THE INVENTION

The present disclosure generally relates to the field of systems and methods for manipulating bacterial growth and abundance in biological sample. More particularly, the present invention relates to systems and methods for manipulating gut microbiota.

### BACKGROUND OF THE INVENTION

Over the recent years many efforts have been invested in understanding the link between gut microbiota and health. It was clearly shown that changing the composition of gut resident micro-organisms might have a profound effect on certain health conditions. Based on the scientific evidences accumulated so far and an ongoing research, it seems that the ability to selectively manage the composition of microorganisms in the GI tract, could have an impact of various diseases and be instrumental in treatment. Moreover, it has been demonstrated that adding specific bacteria as food supplement is insufficient. The entire population of micro-organisms inside the GI tract which comprises wide variety of species, which is referred to as gut microbiota, should be taken into consideration, to make an impact. Thus, the ability to control the composition of the gut microbiota, by enriching specific populations of micro-organisms has strong therapeutic potential. Indeed, maintaining biological diversity in the GI tract has been shown beneficial for various food allergies, inflammatory diseases of the GI tract, diabetes and obesity.

Given the diversity of gut microbiota and the complexity of human GI tract as therapeutic target, there is an unmet need in effective, accessible, easy-to-use therapeutic tools for manipulating human gut microbiota.

### BRIEF DESCRIPTION OF THE DRAWINGS

**Fig.1****:** illustrates a heatmap presenting a profile of gut micro-organisms in a fecal sample;
**Fig.2****:** illustrates Principal Coordinates Analysis (PCoA) presenting microbiota composition;
**Fig.3****:** illustrates Alpha diversity (community richness) rarefaction plots by treatment using Faith's Phylogenetic Diversity estimate;
**Fig.4****:** illustrates Alpha diversity (community richness) rarefaction plots by treatment using observed OTU (Operational Taxonomic Units) estimate;
**Fig.5****:** illustrates boxplots comparing the normalized relative abundance levels of differentially abundant species between treatment samples and controls; and
**Fig.6****:** illustrates boxplots comparing the normalized relative abundance levels of differentially abundant bacteria in the family level between treatment samples and controls.

### SUMMARY OF THE INVENTION

Accordingly, it is a principal object of the present invention to providing an efficient system and method for manipulating human gut microbiota inside and outside the Gastrointestinal (GI) tract and use of such system as potential therapeutic tool. The invention provides a method for manipulating the growth of a plurality of bacterial species of gut microbiota comprising subjecting the plurality of bacterial species to at least one protocol, wherein said protocol comprises a predefined set of conditions, and, wherein said set of conditions comprises at least one episode of illumination at the wavelength in the range of 400 nm to 900nm.

The invention further provides a method for obtaining a sample having a desired profile of bacterial species of gut microbiota, the method comprising:
a) providing a starting sample of bacterial species of gut microbiota;
b) determining the profile of the bacterial species in the starting sample of gut microbiota;
c) selecting at least one protocol designed to manipulate the growth of bacterial species, wherein said protocol comprises a predefined set of conditions, and, wherein said predefined set of conditions comprises at least one illumination episode at the wavelength in the range of 400nm to 900nm;
d) subjecting the starting sample to the at least one protocol; and, optionally,
e) determining the profile of the bacterial species in the sample.

The invention further provides a system for manipulating human gut microbiota *in-situ* comprising:
a) a sample of human microbiota having desired bacterial species profile;
b) a swallowable vehicle configured to move through the GI tract of a human subject, wherein said vehicle comprises at least one light emitting unit, and at least one reservoir configured to carry and release the sample of human microbiota;
c) at least one protocol for manipulating the gut microbiota, wherein said protocol comprises a predefined set of conditions, and, wherein said set of conditions comprises at least one illumination episode at the wavelength in the range of 400nm to 900nm;
d) a software operated controller configured to control the vehicle; and,
e) a software configured to upload the at least one predefined set of conditions to the software operated controller.

The invention yet further provides a method for treating a disorder in a human subject by manipulating gut microbiota, the method comprising:
a) providing a system for manipulating human gut microbiota in-situ, wherein said system comprises a swallowable light emitting vehicle configured to move through the GI tract of a human subject; a software; a software operated controller and a plurality of protocols for manipulating the gut microbiota, wherein each protocol comprises a predefined set of conditions, and, wherein said set of conditions comprises at least one illumination episode at the wavelength in the range of 400nm to 900nm;
b) Selecting at least one of the plurality of protocols for manipulating gut microbiota suitable for treating the disorder;
c) uploading said at least one protocol to the software operated controller of the vehicle;
d) swallowing the vehicle; and
e) delivering the at least one protocol for manipulating gut microbiota at a desired location of the GI tract of the subject.

The invention yet further provides method of fecal transplantation in a subject comprising:
a) preparing a fecal sample suitable for transplantation at the GI tract of the subject
b) providing a swallowable vehicle configured to move through the GI tract of a human subject, wherein said vehicle comprises at least one light emitting unit, a software operated controller, and at least one reservoir configured to carry and release the fecal sample;
c) loading the reservoir with the fecal sample;
d) programming the software operated controller to deliver the fecal sample to a desired location in the GI tract;
e) swallowing the vehicle; and,
f) releasing the fecal sample at the desired location of the GI tract.

Additional features and advantages of the invention will become apparent from the following drawings and description.

### DETAILED DESCRIPTION OF THE INVENTION

Before explaining at least one embodiment of the invention in detail, it is to be understood that the invention is not limited in its application to the details of construction and the arrangement of the components set forth in the following description, examples or illustrated in the drawings. The invention is applicable to other embodiments or of being practiced or carried out in various ways. Also, it is to be understood that the phraseology and terminology employed herein is for the purpose of description and should not be regarded as limiting.

The invention provides a method for manipulating the growth of a plurality of bacterial species of gut microbiota comprising subjecting the plurality of bacterial species to at least one protocol, wherein said protocol comprises a predefined set of conditions, and, wherein said set of conditions comprises at least one episode of illumination at the wavelength in the range of 400 nm to 900nm. As used herein, the term "plurality" refers, without limitation to a large number of different types of microorganisms associated with the gut. The term "plurality", in the context of the invention can be understood as "diversity" of the bacterial species in the gut. The term "gut microbiota" as used herein, is meant to be understood without limitation as a total of micro-organisms residing and/or associated with the GI tract or the gut in healthy and/or diseased state. According to some embodiments, manipulating the growth of the plurality of bacterial species of gut microbiota comprises obtaining a sample of gut microbiota from a subject and, subjecting said sample to the at least one protocol comprising at least one episode of illumination at the wavelength in the range of 400nm to 900nm. According to some embodiments, manipulating the growth of the plurality of bacterial species of gut microbiota is done *in-situ* and comprises subjecting at least a portion of GI tract of a subject to the at least one protocol comprising at least one episode of illumination at the wavelength in the range of 400nm to 900nm. The phrase "at least a portion of GI tract" is meant to be understood, without limitation, as a region or a part either small part or a large part, including, without limitation, stomach, small or large intestine of the gastrointestinal tract of the subject. To avoid any doubt, more than one portion of the GI tract can be subjected to the method of the invention. In one embodiment, manipulating the growth of the plurality of bacterial species of gut microbiota is done *in-situ* and comprises subjecting more than one portion of GI tract of the subject to the at least one protocol comprising at least one episode of illumination at the wavelength in the range of 400nm to 900nm. In another embodiment, a single portion of the GI tract is subjected to one or more protocols. In one embodiment, a single portion of the GI tract is repeatedly subjected to a single protocol. In yet another embodiment, a single portion of the GI tract is subjected to multiple protocols. According to some embodiments, a portion and/or portions of the GI tract of the subject may be subjected to one and/or multiple protocols, in any order or any combination suitable to achieve the desired effect. As used herein, the term "manipulating" refers, without limitation, to influencing the composition of bacterial species populations. In one embodiment, the term "manipulating" refers to inhibiting the growth of at least one of the plurality of the bacterial species. In another embodiment, the term "manipulating" refers to eliminating at least one bacterial species in the sample. In yet another embodiment, the term "manipulating" refers to enhancing the growth of at least one bacterial species in the sample. As used herein, the term "protocol" refers, without limitation, to a predetermined set of conditions combination of which is designed to lead to a desired outcome. In one embodiment, the protocol comprises at least one of the following: comprises at least one of the parameters selected from the group consisting of illumination doses, wavelengths, duration of each illumination, number of illumination episodes, frequency of illumination episodes, exposure to oxygen, humidity, exposure to acoustic wave, pH, exposure to radio frequency, temperature, presence of medication, presence of bacterial species, presence of bioactive substance, or any combination thereof. As used herein, the term "bioactive substance" refers, without limitation to signaling molecules, biomolecules; genetic and translation modifying nucleic material; deoxyribonucleic acids (DNA); ribonucleic acids (RNA); organic molecules; inorganic molecules; amino acids; vitamins; polyphenol, steroid, lipophilic poor soluble drug, vasomodulators; peptides; neurotransmitters and analogues of thereof; nucleosides; proteins, including without limitation growth factors, hormones, aptamers, antibodies, cytokines, enzymes, and heat shock proteins; cannabinoid; cannabinoid acid such as tetrahydrocannabinolic acid (THCa), cannabidiolic acid (CBDa), cannabinolic acid (CBNa) cannabichromenic acid (CBCa), tetrahydrocannabinol (THC), cannabinol (CBN), cannabidiol (CBD), and cannabichromene (CBC), and endocannobinoids and their analogues, or any other molecules that can exert biological function. As used herein, the term "medication" refers to a substance having a therapeutic effect. As used herein, the term "therapeutic effect" refers, without limitation to response(s) after a treatment of any kind, the results of which are judged to be useful or favorable. This is true whether the result was expected, unexpected, or even an unintended consequence. In one embodiment, the therapeutic effect is selected from the group consisting of anti-inflammatory effect, anti-fibrotic effect, anti-tumor effect, and neuroprotective effect. A non-limiting medicines for combating inflammation, fibrosis, hyperglycolisis, such as diabetic nephropathy, comprises naturally derived ubiquitous phenolic compounds, such as ferulic acid, natural phenols, for example resveratrol, rutin, quercetin, phenolic acids, vitamins, and allicin, cannabinoids selected from the group consisting of tetrahydrocannabinolic acid (THCa), cannabidiolic acid (CBDa), cannabinolic acid (CBNa) cannabichromenic acid (CBCa), tetrahydrocannabinol (THC), cannabinol (CBN), cannabidiol (CBD) and cannabichromene (CBC)or/ and the derivatives of thereof , and synthetic analogues, dexmedetomidine, (α2-AR) agonist, metabolic protectants, including, without limitation, vildagliptin, pristimerin, metformin , pyridoxamine, vasomodulators, epinephrine, rutin, isoxsuprine. The non-limiting list medicines exerting anti-tumor effect comprises, without limitation, chemotherapeutic agent, including without limitation cisplatin, carboplatin, chlorambucil, melphalan, nedaplatin, oxaliplatin, triplatin tetranitrate, satraplatin, imatinib, nilotinib, dasatinib, and radicicol; an immunomodulatory agent, an antiangiogenic agent, a mitotic inhibitor, a nucleoside analog, a DNA intercalating agent, anti-ageing agents, dipeptides, epigenetic factors and modulators, metformin, rapamycin, valproic acid or salt, wortmannin, polyamine spermidine, HDAC inhibitors sodium butyrate, butyric acid, sirtuin activators, resveratrol, coenzyme CoQ1, small dicarboxylic acids, aspirin, salicylic, benzoic acid, carnitine analogues, human growth hormone, a topoisomerase analogue, an antibody, a cytokine, a folate antimetabolite, anti-glycolisis agent, inhibitor oligonucleotide to a human oncogenic or proto-oncogenic transcription factor, chemotherapeutic agent, an immunomodulatory agent, an antiangiogenic agent, a mitotic inhibitor, a nucleoside analogue, a DNA intercalating agent, a topoisomerase analog, an antibody, a cytokine, or a folate antimetabolite, hexokinase inhibitor, a lactate dehydrogenase inhibitor, a phosphofructokinase 2 or phosphofructo-2-kinase/fructose-2,6-bisphosphatase inhibitor, a pyruvate kinase M2 inhibitor, a transketolase inhibitor, a pyruvate dehydrogenase inhibitor, a pyruvate dehydrogenase kinase inhibitor, a glucose-6-phosphate dehydrogenase inhibitor, a GLUT inhibitor, a proton transport inhibitor, a monocarboxylate transporter inhibitor, a hypoxia-inducible factor 1 alpha inhibitor, an AMP-activated protein kinase inhibitor, a glutamine inhibitor, an asparagine inhibitor, an arginine inhibitor, a fatty acid synthase inhibitor, an ATP-citrate lyase inhibitor, dimethyl malate, and malic enzyme 2 inhibitor, or any combination thereof. As used herein, the term "sample of gut microbiota" refers, without limitation to a natural sample originating from GI tract of a human subject. The sample of the invention may be collected directly from the GI tract of the subject or from body waste excreted from the body, such as feces. In one embodiment, the protocol comprises at least one episode of illuminating the sample at the wavelength in the range of 400nm to 900nm. In one embodiment, the protocol comprises at least one episode of illuminating the sample at the wavelength in the range of 420nm to 880nm. In one embodiment, the wavelength is 420-460nm; 640-680nm; and 830-870nm. According to some embodiments, the wavelength 400nm, 410nm, 420nm, 430nm, 440nm, 450nm, 460nm, 470nm, 480nm, 490nm, 500nm, 510nm, 520nm, 530nm, 540nm, 550nm, 560nm, 570nm, 580nm, 590nm, 600nm, 610nm, 620nm, 630nm, 640nm, 650nm, 660nm, 670nm, 680nm, 690nm, 700nm, 710nm, 720nm, 730nm, 740nm, 750nm, 760nm, 770nm, 780nm, 790nm, 800nm, 810nm, 820nm, 830nm, 840nm, 850nm, 860nm, 870nm, 880nm, 890nm,and 900nm. In one embodiment, the protocol comprises at least two illumination episodes. In yet another embodiment, the protocol comprises a sequence of illumination episodes. In another embodiment, similar wavelength is used in each illumination episode. In yet further embodiment, at least two illumination episodes are carried out at different wavelengths. For the sake of clarity, the protocol of the invention may involve as many illumination episodes as needed, at different or identical wavelengths and for the desired duration of time.

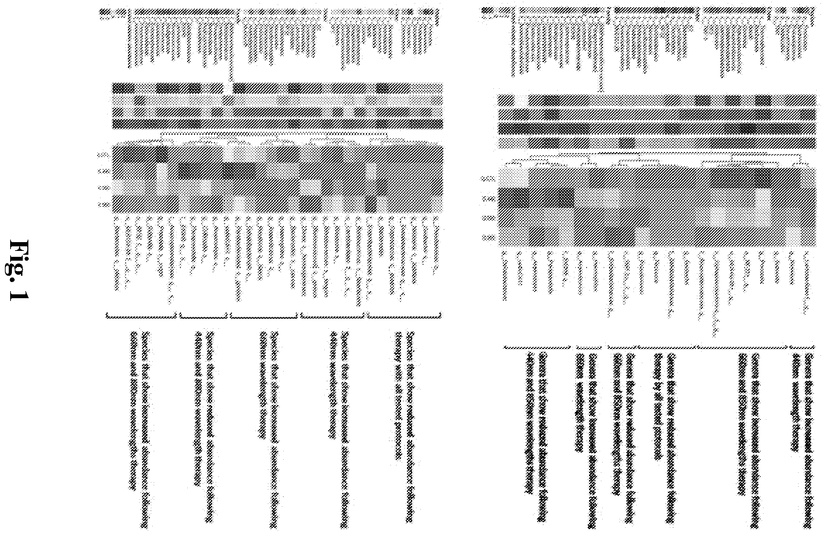

Any combination of illumination at the indicated wavelengths with other parameters constitute an integral part of the present invention. In one embodiment, the protocol is designed to generate a specific profile of a plurality of bacterial species in the sample. Reference is now made to Fig. 1 illustrating an exemplary embodiment of a heatmap presenting a profile of gut micro-organisms in a fecal sample at the genus level following illumination episodes with respect to the wavelength. Horizontal lines represent the relative abundance of major genera that exhibited at least 50% change relative to control. The bacterial abundance of each genus is expressed in standard deviation units scaled internally on a row basis and represented by color key. Hierarchical clustering was based on Euclidian distance measure and Word's agglomeration method. Side bars show the taxonomic classification of the different genera in the phylum, class, order and family level. A non-limiting list of gut micro-organisms in the sample comprises: bacterial species of *Roseburia genus; Sutterella* genus; *Prevotella* genus; *Haemophilus genus; Anaerotruncus* genus; *Geobacter* genus; *Neisseria* genus; *Pseudomonas* genus; *Paraprevotella* genus; *Anaerostipes* genus; *Dehalobacterium* genus; *Coprococcus eutactus; Ruminococcus* genus; *Bifidobacterium longum; Dorea formicigenerans; Bacteroides caccae;* and *Parabacteroides gordonii.* For the sake of clarity, and to avoid any doubt, any gut microorganism is an integral part of the present invention. According to some embodiments, the genus *Roseburia,* which consists of obligate gram-positive anaerobic bacterial species and includes five species: *R. intestinalis, R. hominis, R. inulinivorans, R. faecis and R. cecicola,* was increased in 440nm treatment. According to some embodiments, the genus *Sutterella,* which represents gram negative anaerobic bacteria and includes three species: *S. parvirubra, S. stercoricanis and S. wadsworthensis* was increased in 660nm and 880nm treatments. According to some embodiments, the genus *Prevotella* which includes almost 40 different species with a large genomic diversity was increased in 660nm and 880nm treatments. According to some embodiments, the *Geobacter* species, *Neisseria* genus, and *Pseudomonas* genus, were reduced under all illumination treatments.

In one embodiment, the protocol is designed to enrich at least one microorganism genus in-vitro and/or in-vivo and/or in-situ. As used herein, the term "to enrich" refers, without limitation, to an increase in the proportion of a particular bacterial genus and/or species in the sample. In one embodiment, the protocol is designed to enrich at least one bacterial species. In one embodiment, the protocol is designed to enrich at least one bacterial genus. In one embodiment, the protocol is designed to increase the growth of at least one bacterial species and/or genus and to inhibit the growth of at least one bacterial species and/or genus at the same time. In yet another embodiment, the protocol is designed to increase the growth of more than one bacterial species and/or genii and to inhibit growth of more than one bacterial species and/or genii at the same time. In one embodiment, the protocol is designed to eliminate at least one bacterial species and/or genii. In one embodiment, the protocol is designed to downsize at least one bacterial species and/or genii. In one embodiment, the protocol is designed to obtain a human gut microbiota sample having a desired profile of bacterial species and/or genii. In one embodiment, the protocol. In one embodiment of the invention, the protocol is designed to selectively enhance, reduce or eliminate microorganism species from the sample to obtain a human gut microbiota sample having a desired profile. As used herein, the term "profile" refers, without limitation, to an information relating to characteristics of the sample based on quantification of bacterial species populations constituting the gut microbiota sample.

The invention further provides A method for obtaining a sample having a desired profile of bacterial species of gut microbiota, the method comprising:
a) providing a starting sample of bacterial species of gut microbiota;
b) determining the profile of the bacterial species in the starting sample of gut microbiota;
c) selecting at least one protocol designed to manipulate the growth of bacterial species, wherein said protocol comprises a predefined set of conditions, and, wherein said predefined set of conditions comprises at least one illumination episode at the wavelength in the range of 400nm to 900nm;
d) subjecting the starting sample to the at least one protocol; and, optionally,
e) determining the profile of the bacterial species in the sample.

In one embodiment, the at least one protocol comprises at least two illumination episodes. In another embodiment, the at least one protocol comprises a sequence of multiple illumination episodes. In one embodiment, the wavelength of illumination is similar in each episode. In one embodiment, the wavelength of illumination is similar in each episode. In one embodiment, the wavelength of illumination is similar in each episode. In another embodiment, the illumination episodes are carried out at least two different wavelengths. In one embodiment, the at least one protocol is designed to enhance the growth of at least one bacterial species population in the starting sample. In another embodiment, the at least one protocol is designed to inhibit the growth of at least one bacterial species population in the starting sample. In another embodiment, the at least one protocol is designed to eliminate the growth of at least one bacterial species population in the starting sample. In one embodiment, the starting sample subjected to at least two protocols. In another embodiment, the at least two protocols are designed to manipulate growth of different bacterial species populations in the starting sample. In one embodiment, the wavelength is 420-460 nm; 640-680 nm; and 830-870nm. According to some embodiments, the wavelength 400nm, 410nm, 420nm, 430nm, 440nm, 450nm, 460nm, 470nm, 480nm, 490nm, 500nm, 510nm, 520nm, 530nm, 540nm, 550nm, 560nm, 570nm, 580nm, 590nm, 600nm, 610nm, 620nm, 630nm, 640nm, 650nm, 660nm, 670nm, 680nm, 690nm, 700nm, 710nm, 720nm, 730nm, 740nm, 750nm, 760nm, 770nm, 780nm, 790nm, 800nm, 810nm, 820nm, 830nm, 840nm, 850nm, 860nm, 870nm, 880nm, 890nm,and 900nm.

In one embodiment, the at least one protocol further comprises at least one of the parameters selected from the group consisting of illumination doses, wavelengths, duration of each illumination, number of illumination episodes, frequency of illumination episodes, exposure to oxygen, humidity, exposure to acoustic wave, pH, exposure to radio frequency, temperature, presence of medication, presence of bacterial species, and presence of bioactive substance.

According to some embodiments, the invention provides a system for manipulating human gut microbiota in-situ comprising:
a) a sample of human microbiota having desired bacterial species profile;
b) a swallowable vehicle configured to move through the GI tract of a human subject, wherein said vehicle comprises at least one light emitting unit, and at least one reservoir configured to carry and release the sample of human microbiota;
c) at least one protocol for manipulating the gut microbiota, wherein said protocol comprises a predefined set of conditions, and, wherein said set of conditions comprises at least one illumination episode at the wavelength in the range of 400nm to 900nm;
d) a software operated controller configured to control the vehicle; and,
e) a software configured to upload the at least one predefined set of conditions to the software operated controller.

In one embodiment, the software operated controller is configured to fulfill at least one of the following functions: regulating the function of the light emitting unit, regulating the motion of the vehicle, identifying the location of the vehicle, instructing the vehicle to reach a desired location of the GI tract; delivering the at least one protocol for manipulating the gut microbiota at the desired location, and, actuating release of the cargo from the at least one reservoir at the desired location. comprises a plurality of light emitting elements configured to emit a light at the wavelength in the range of 400nm to 900nm. In one embodiment, the light emitting element is a light emitting diode (LED) or a laser beam. In one embodiment, the light emitting element is light emitting diode (LED). According to some embodiments, the system further comprises at least one thermo-electric element in communication with the controller, wherein said thermo-electric element is configured to control local temperature. In one embodiment, the at least one thermo-electric element configured to increase or decrease the local temperature. In another embodiment, the swallowable vehicle comprises at least two thermo-electric elements. In one embodiment the swallowable vehicle of the invention further comprises a second reservoir in communication with the controller and loaded with a liquid media and designed to release the liquid media in a desired location of the GI tract. In one embodiment, release of the liquid media from the second reservoir at the desired location results in a change in humidity. In one embodiment, the liquid media is water or saline. In one embodiment, the reservoir comprises a dispenser.

According to some embodiments, the controller is configured to deliver two or more protocols for manipulating gut microbiota at the desired location. In one embodiment, the two or more protocols are delivered at the same location of the GI tract of the human subject. In yet another embodiment, the two or more protocols are delivered at different locations of the GI tract. In one embodiment, the controller is configured to deliver a plurality of protocols. In one embodiment, the plurality of protocols is delivered at different locations of the GI tract of the subject.

The invention further provides a method for treating a disorder in a human subject by manipulating gut microbiota, the method comprising:
a) providing a system for manipulating human gut microbiota in-situ, wherein said system comprises a swallowable light emitting vehicle configured to move through the GI tract of a human subject; a software; a software operated controller and a plurality of protocols for manipulating the gut microbiota, wherein each protocol comprises a predefined set of conditions, and, wherein said set of conditions comprises at least one illumination episode at the wavelength in the range of 400nm to 900nm;
b) Selecting at least one of the plurality of protocols for manipulating gut microbiota suitable for treating the disorder;
c) uploading said at least one protocol to the software operated controller of the vehicle;
d) swallowing the vehicle; and
e) delivering the at least one protocol for manipulating gut microbiota at a desired location of the GI tract of the subject.

In one embodiment, the swallowable vehicle comprises at least one reservoir configured to carry and release a cargo. In another embodiment, further comprising the step of loading a sample derived from human gut microbiota having a desired profile of bacterial species to the at least one reservoir. In another embodiment, the step of loading the sample derived from gut microbiota to the reservoir precedes step d. In yet another embodiment, the method further comprising the step of releasing the sample derived from human gut microbiota or a portion thereof at the desired location of the GI tract of the subject. In one embodiment, the step of releasing the sample precedes step e. In another embodiment, the step of releasing the sample follows step e. In one embodiment, the protocol comprises multiple illumination episodes. In one embodiment, the software operated controller is configured to fulfill at least one of the following functions: regulating the function of the light emitting unit, regulating the motion of the vehicle, identifying the location of the vehicle, instructing the vehicle to reach a desired location of the GI tract; delivering the at least one protocol for manipulating the gut microbiota at the desired location, and, actuating release of the cargo from the at least one reservoir at the desired location. In yet another embodiment, the protocol further comprises at least one of the parameters selected from the group consisting of illumination doses, wavelengths, duration of each illumination, number of illumination episodes, frequency of illumination episodes, exposure to oxygen, humidity, exposure to acoustic wave, pH, exposure to radio frequency, temperature, presence of medication, presence of bacterial species, and presence of bioactive substance. According to some embodiments, the non-limiting list of disorders of the invention includes: Inflammatory Bowel Diseases, Crohn Disease, Type II Diabetes, HIV, malignancy of GI tract, Ulcerative Colitis, Proctitis, Over-weight, Irritable Bowel syndrome, Alzheimer, depression, Celiac, Specific food allergies, Clostridium Difficile infection, Diarrhea, Chronic constipation, and Dysbiosis.

The invention further provides a method of fecal transplantation in a subject comprising:
a) preparing a fecal sample suitable for transplantation at the GI tract of the subject;
b) providing a swallowable vehicle configured to move through the GI tract of a human subject, wherein said vehicle comprises at least one light emitting unit, a software operated controller, and at least one reservoir configured to carry and release the fecal sample;
c) loading the reservoir with the fecal sample;
d) programming the software operated controller to deliver the fecal sample to a desired location in the GI tract;
e) swallowing the vehicle; and,
f) releasing the fecal sample at the desired location of the GI tract.

As used herein, the term "preparing a fecal sample" refers, without limitation, to subjecting the fecal sample to at least one protocol of the invention to obtain a sample having desired profile of bacterial species. In one embodiment, the method of fecal transplantation further comprises a step of delivery of at least one protocol for manipulating gut microbiota. In one embodiment, the protocol comprises at least one episode of illuminating the GI tract of the subject at the wavelength in the range of 400nm to 900nm. In one embodiment, the protocol comprises multiple illuminating episodes. According to one embodiment, the protocol comprises at least one of the parameters selected from the group consisting of illumination doses, wavelengths, duration of each illumination, number of illumination episodes, frequency of illumination episodes, exposure to oxygen, humidity, exposure to acoustic wave, pH, exposure to radio frequency, temperature, presence of medication, presence of bacterial species, presence of bioactive substance, or a combination thereof.

According to some embodiments, the subject is afflicted with a disorder characterized by impaired gut microbiota. A non-limiting list of disorders characterized by impaired gut microbiota includes: Inflammatory Bowel Diseases, Crohn Disease, Type II Diabetes, HIV, malignancy of GI tract, Ulcerative Colitis, Proctitis, Over-weight, Irritable Bowel syndrome, Alzheimer, depression, Celiac, Specific food allergies, Clostridium Difficile infection, Diarrhea, Chronic constipation, and Dysbiosis.

### Example 1: Effect of the tested low-level light therapy (LLLT) on the fecal microbiome composition using several illumination regimens.

The objective of the study was to test the effect of three different wavelengths of low-level light therapy on fecal microbiota composition *in-vitro.*

The study was performed using three experimental frameworks:
1. Long term illumination exposure of the different tested regimens to assess microbial composition change following treatment.
2. Short term experiment combining bacterial composition assessment and inflammatory related function.
3. *C. difficile* toxicogenic strain experiment, to evaluate the specific effect of LLLT on the bacterium growth.

### Framework 1: Long term experiment with longitudinal illumination exposure

The tested regimens with regard to the Illumination wavelengths and exposure times are detailed in table 2. Illumination was performed in 5 exposures, where in each day, 2 exposures were performed (same time every day, at least 8 hours interval between exposures, i.e. 8:00 am and 16:00 pm) for 2.5 days.

**Table 2: The different tested illumination regimens***

| **Regimen** | **Wavelength** | **Exposure time (sec)** |
|---|---|---|
| **1** | 440nm | 50 |
| **2** | 660nm | 60 |
| **3** | 850mm | 110 |
| **4** | control | |

| | | |
|---|---|---|
| Sample size: 10 fecal samples were tested for each regimen. A total of 40 tested samples + original pre-plated uncultured sample. | | |

### The experimental procedure:

**1.** Preparation of the fecal sample
   a. The fecal sample were collected (preferentially from individuals that were not treated with antibiotics in the last year) in sterile specimen and immediately were frozen in -20°C. While being transported to the lab, the stool samples were retained in anaerobic jar with gas pack, within a container with ice or dry ice
   b. In the lab, the samples were be stored in -80°C.
**2.** Culturing of fecal sample
   a. For culturing, samples were placed in anaerobic chamber and be adjusted to room temperature.
   b. Inside an anaerobic chamber, 0.2 g of fecal sample was diluted in 1800µl of pre-reduced liquid medium (BHI with supplements).
   c. The fecal material was suspended by vortexing for 5 min.
   d. The fecal suspension was standing at room temperature for 5min to allow the precipitation of insoluble ingredients.
   e. Then 50 µl of the suspension were plated on the agar plates (size 35 mm) followed by incubation in anaerobic conditions at 37°C for 3 days in which they were exposed to the illumination treatment twice a day as detailed above.
   f. Samples were incubated in a dark place between illumination exposures.
   g. 500 µl of the starting fecal suspension were stored as a frozen stock at -80°C , by the addition of glycerol to reach a final concentration of 25% glycerol (equal volume of bacterial suspension in growth media, and 1:1 pre-reduced glycerol with PBS) for optional bacterial composition assessment.
**3.** Illumination treatment
   Illumination was performed in 5 exposures, where in each day, 2 exposures were performed (same time every day, at least 8hr. interval between exposures, i.e. 8:00 am and 16:00 pm) for 2.5 days within the anaerobic chamber. After the 5^{th} exposure, a 6-hour duration between illumination and bacterial harvesting is required. Illumination device was held in a specific pre-defined distance from the plate (using a special holder), in order to equally expose all the plate surface.
**4.** Bacterial harvesting at end point
   a. 0.5 ml of liquid media (BHI) were added to each plate and bacterial colonies were scraped and suspended in the liquid medium.
   b. 250 µl of the harvested bacteria, were added to equal volume of 1:1 glycerol with PBS (25% glycerol final conc.) and were frozen at -80°c for RNA extraction.
   c. The rest of the fecal suspension were collected and stored in the same manner as detailed in stage 4.b for the next experiment of co-culture.

### Framework 2: short term experiment with assessment of bacterial-host function post treatment

To isolate the direct effect of the illumination treated fecal microbiota, effects of non-treated and treated fecal microbiota (obtained in experiment 1) on epithelial cells using co-cultures were tested.
1. Pre-growth of HT-29 cells:
   a. 1ml of thawed HT-29 stock at a concentration of 3-4*10⁶ cells/ml were suspended in 9ml pre warmed McCoy medium supplemented with10 % (v/v) inactivated FCS (fetal calf serum) and 2mM glutamine to wash the DMSO.
   b. Then cells were centrifuged at 125g, at room temperature, for 10 min.
   c. The medium were aspirated and suspended in 10ml McCoy medium supplemented with10 % (v/v) FCS and 2mM glutamine.
   d. Cells were plated in T-25 cm2 flask. The cells were incubated in 37°C for 2-4 days in 5 % CO2 air atmosphere.
   e. The cells were harvested when reaching 80-90 % confluent growth as follows: cells were detached from the flask by aspiration of the medium and the addition of 1ml trypsin (0.25% w/v trypsin-0.53mM EDTA) followed by incubation for 5min in 37°C. Cells detachment was visualized under microscope.
   f. To stop the trypsin activity 1ml of McCoy medium supplemented with10 % (v/v) FCS and 2mM glutamine were added.
   g. Then medium was added accordingly to obtain a final concentration of 5×10⁶ cells/ml.
2. Co-culture
   a. HT29 colonic epithelial cells were seeded at a density of 5×10⁴ cells/cm² onto 24-well plates in 1ml McCoy medium supplemented with 10% inactivated FCS and 2mM glutamine and grown at 37°C in 5% CO₂ and 95% air for 24 hr.
   b. For co-culture, the medium were changed to reduced media and 100µl of fecal bacterial suspensions from experiment 1 were added to each well of a 24-well plate.
   c. Co-cultures were incubated at 37°C for 6 hrs. under anaerobic conditions.
   d. At experiment end-point:
      a) The bacterial cell suspension was collected from each well (the HT29 will be attached to the bottom). 500µl of the bacteria suspension was added to equal volume of 1:1 glycerol with PBS (25% glycerol final conc.) and will be frozen at -80°c for RNA extraction.
      b) Then, 350µl cell lysis reagent (RLT buffer+ beta mercaptoethanol) that was used in the first step of RNA extraction procedure (QIAGEN) was added to each well. Cells were scraped from the well in order to be transferred to the lysis buffer.
      c) Next extraction steps were followed immediately according to the manufacturer's instruction. The extracted RNA will be preserved in -80°C for assessment of inflammatory cytokine expression using real time PCR.

### Framework 3: The effect of illumination treatment on the growth of toxigenic C.difficile.

### C.difficile strain selection:

### Strain:

Toxigenic strain of *C.difficile* that expresses both the tcdA and tcdB genes required for the production of toxin A and toxin B: *Clostridioides difficile* (Prevot) Lawson et al. (ATCC^{®} 43255^{™}) that belongs to toxinotype 0 was used.

*C.difficile* inhibition was compared to a reference beneficial species such as *Bifidobacterium longum* that was reported to reduce mucosal inflammation marker in IBD (can be grown on MRS at 37°C anaerobically) ³
1. Recovery of *C.difficle* stock was performed according to the ATCC instructions.
2. *C. difficile* was plated on brain heart infusion broth supplemented with yeast extract (0.5%, wt/vol) and l-cysteine (10%, wt/vol) (BHIS -Brain heart infusion-supplemented), and were incubated at 37°C under anaerobic condition for 48 h, as previously reported^{4,5}.
3. Illumination treatment:
a. *C.difficile* culture was adjusted to an optical density of 0.5 at 600 nm (OD₆₀₀) (mid exponential phase).
b. The adjusted culture was split into 4 sample falcons, each falcon for each tested illumination treatment.
c. The adjusted culture was poured into a sterile dish with high surface area to obtain a low bacterial suspension height (about 0.5 cm) under aseptic conditions in anaerobic chamber.
d. Illumination exposure was performed as detailed in the table below (single treatment with different duration exposures is recommended if possible):

**Table 3: The different tested illumination treatments in the C.difficile experiment**

| **Regimen** | **Wavelength** | **Exposure time (sec)** |
|---|---|---|
| **1** | 440nm | 50 |
| **2** | 660nm | 60 |
| **3** | 850nm | 110 |
| **4** | control | |

a. Optical density (at 600 nm) of *C*. *difficile* cultures after illumination treatments and control were measured periodically during anaerobic cultivation for 48hr at 37°C to evaluate growth (using plate reader if available).
b. 0, 12, 24, 36, 48 hr post illumination exposure, 500µl of *C.difficile* cultures were used serial dilutions and plating (100µl) on BHIS agar plates and parallel measurement of OD, for the evaluation of CFU (colony forming units) ml⁻¹. Plates will be incubated anaerobically in 37°C for 48hr. The CFU/ml/OD₆₀₀ were determined and extrapolated to the other OD measurements for CFU quantification based on OD.

### Results:

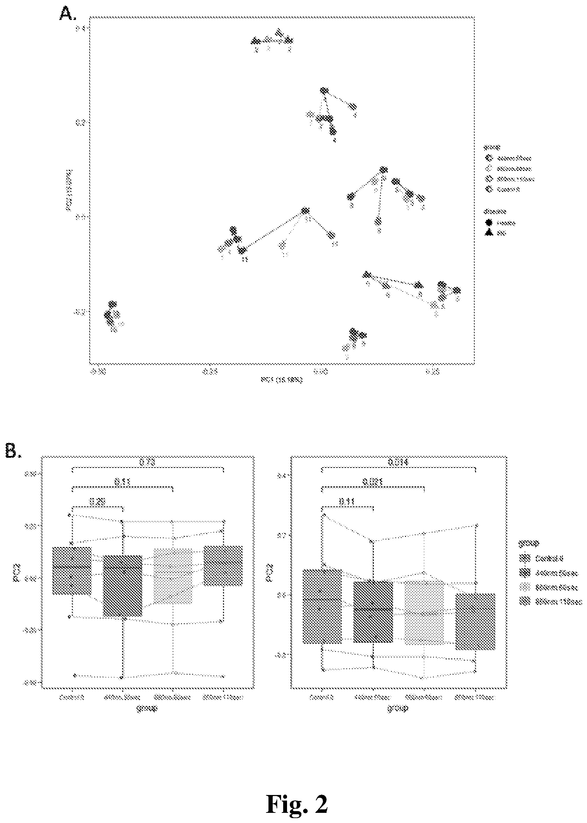

Fig.2 demonstrates that the treatment (660nm & 850nm) induces significant alternations in microbiota composition compared to the control. (A) PCoA (principal coordinates analysis) presenting microbiota composition was used to assess microbial composition dissimilarity based on Bray-Curtis distance. Each point represents the bacterial composition profile of the different tested treatment protocols: 440nm wavelength (blue); 660 nm (orange); 850nm (red) and untreated sample as a control (dark green). Numbers indicated different fecal donors. (B) Boxplots comparing PC1 and PC2 coordinates between the different treatments. The results demonstrate that the treatment (660nm & 850nm) induces significant alternations in microbiota composition compared to the control, as indicated from the significant difference in PC2 coordinates values.

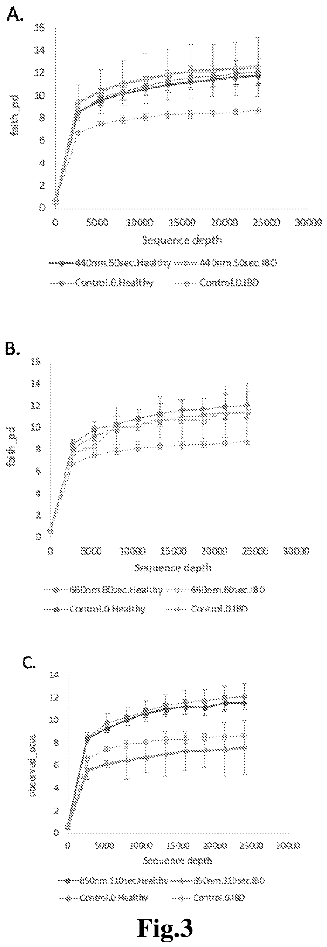

Fig. 3 demonstrates that the treatment increases the microbial diversity of IBD samples using alpha diversity (community richness) rarefaction plots by treatment. Microbial alpha diversity was assessed using rarefaction plots to compare samples from the different treatments and control using the Faith's Phylogenetic Diversity estimate. Error bars correspond to variations between samples of each treatment group. (A)440nm treatment induces increase in microbial diversity in IBD samples. (B) 660nm treatment induces increase in microbial diversity in IBD samples. (C) 850nm treatment has no significant effect on alpha diversity. Healthy samples were not affected due to already high baseline diversity. The results indicate that the 440nm and the 660nm treatments induce increase in the microbial diversity of samples from IBD patients to similar level of samples from healthy donors.

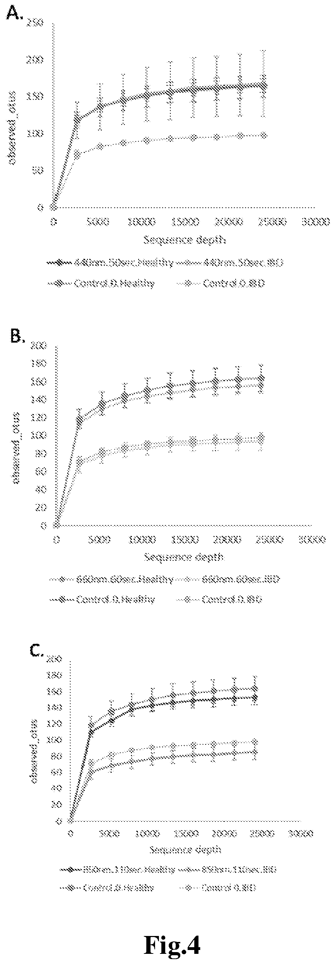

Fig.4 demonstrates that the treatment increases the microbial diversity of IBD samples. Microbial alpha diversity was assessed using rarefaction plots to compare samples from the different treatments and control using the observed OTU (Operational Taxonomic Units) estimate, which represent the count of unique OTUs in each sample. Error bars correspond to variations between samples of each treatment group. (A) 440nm treatment induces increase in microbial diversity in IBD samples. (B) 660nm treatment has no significant effect on alpha diversity. (C) 850nm treatment has no significant effect on alpha diversity.

Healthy samples are not affected due to already high baseline diversity. The results indicate that the 440nm and the 660nm treatments induce increase in the microbial diversity of samples from IBD patients to similar level of samples from healthy donors.

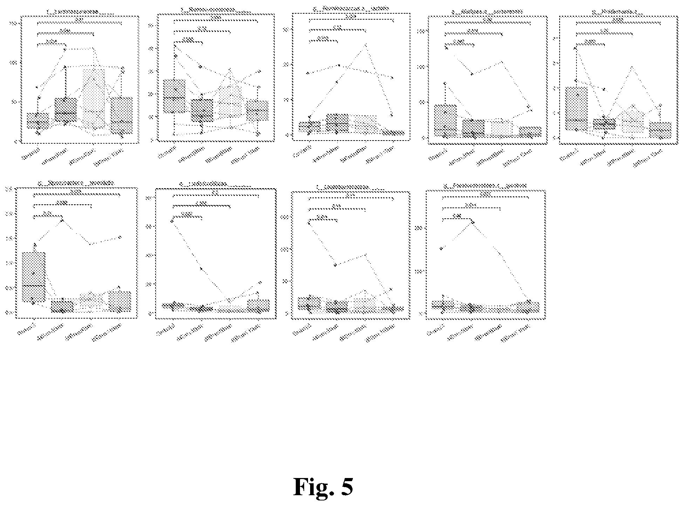

Fig.5 illustrates treatment differentially abundant species. Boxplots comparing the normalized relative abundance levels of differentially abundant species between treatment samples and controls. The normalization was performed by first calculation of relative abundance of each species within each sample multiply by factor 1000. Only samples that had non-zero counts in the control untreated group, in at least 30% of the samples were included. Differential abundance was estimated using paired Wilcoxon test between treated samples vs controls. The results demonstrate a significant increase in the *Lachnospiraceae* sp. and *Ruminococcus lactaris* abundances following 440nm and 660nm treatments. *Lachnospiraceae* and *Ruminococcaceae* were known to be positively associated with healthy individuals. Some species of the *Lachnospiraceae* family are butyrate producers; butyrate production in the human gut is highly relevant as it promotes Treg cell differentiation which can ultimately suppress pro-inflammatory responses.

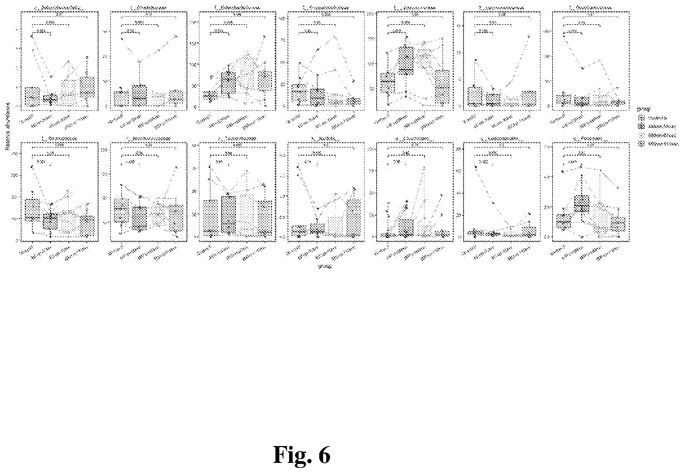

Fig.6 illustrates treatment differential abundance - Family level. Boxplots comparing the normalized relative abundance levels of differentially abundant bacteria in the family level between treatment samples and controls. The normalization was performed by first calculation of relative abundance of each species within each sample multiply by factor 1000. Only samples that had non-zero counts in the control untreated group, in at least 30% of the samples were included. Differential abundance was estimated using paired Wilcoxon test between treated samples vs controls. The results indicate a significant increase in the abundance of the *Lachnospiraceae* family following 440nm and 660nm treatments. *Lachnospiraceae* are important short-chain fatty acids producers.

The terminology used herein is for the purpose of describing particular embodiments only and is not intended to be limiting of the invention. As used herein, the singular forms "a," "an" and "the" are intended to include plural forms as well, unless the context clearly indicates otherwise. It will be further understood that the terms "comprises" or "comprising," when used in this specification, specify the presence of stated features, integers, steps, operations, elements components and/or groups or combinations thereof, but do not preclude the presence or addition of one or more other features, integers, steps, operations, elements, components and/or groups or combinations thereof. As used herein the terms "comprises", "comprising", "includes", "including", "having" and their conjugates mean "including but not limited to". The term "consisting of" means "including and limited to".

As used herein, the term "and/or" includes any and all possible combinations or one or more of the associated listed items, as well as the lack of combinations when interpreted in the alternative ("or").

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and claims and should not be interpreted in an idealized or overly formal sense unless expressly so defined herein. Well-known functions or constructions may not be described in detail for brevity and/or clarity.

It will be understood that when an element is referred to as being "on," "attached" to, "operatively coupled" to, "operatively linked" to, "operatively engaged" with, "connected" to, "coupled" with, "contacting," etc., another element, it can be directly on, attached to, connected to, operatively coupled to, operatively engaged with, coupled with and/or contacting the other element or intervening elements can also be present. In contrast, when an element is referred to as being "directly contacting" another element, there are no intervening elements present.

It will be understood that, terms such as, for example, "processing", "computing", "calculating", "determining", "establishing", "analyzing", "checking", or the like, may refer to operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium that may store instructions to perform operations and/or processes. It will be understood that, although the terms first, second, etc., may be used herein to describe various elements, components, regions, layers and/or sections, these elements, components, regions, layers and/or sections should not be limited by these terms. Rather, these terms are only used to distinguish one element, component, region, layer and/or section, from another element, component, region, layer and/or section.

Certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention.

Certain features described in the context of various embodiments are not to be considered essential features of those embodiments, unless the embodiment is inoperative without those elements.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Whenever terms "plurality" and "a plurality" are used it is meant to include, for example, "multiple" or "two or more". The terms "plurality" or "a plurality" may be used throughout the specification to describe two or more components, devices, elements, units, parameters, or the like. The term set when used herein may include one or more items. Unless explicitly stated, the method embodiments described herein are not constrained to a particular order or sequence. Additionally, some of the described method embodiments or elements thereof can occur or be performed simultaneously, at the same point in time, or concurrently.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

By "patient" or "subject" is meant to include any mammal. A "mammal," as used herein, refers to any animal classified as a mammal, including but not limited to, humans, experimental animals including monkeys, rats, mice, and guinea pigs, domestic and farm animals, and zoo, sports, or pet animals, such as dogs, horses, cats, cows, and the like.

"Treating" or "treatment" of a disease as used herein includes: preventing the disease, i.e. causing the clinical symptoms of the disease not to develop in a mammal that may be exposed to or predisposed to the disease but does not yet experience or display symptoms of the disease; inhibiting the disease, i.e., arresting or reducing the development of the disease or its clinical symptoms, or relieving the disease, i.e., causing regression of the disease or its clinical symptoms.

In case of conflict, the patent specification, including definitions, will prevail. In addition, the materials, methods, and examples are illustrative only and not intended to be limiting. Throughout this application various publications, published patent applications and published patents are referenced.

It will be appreciated by persons skilled in the art that the present invention is not limited to what has been particularly shown and described hereinabove. Rather the scope of the present invention is defined by the appended claims and includes both combinations and sub-combinations of the various features described hereinabove as well as variations and modifications thereof, which would occur to persons skilled in the art upon reading the foregoing description.

## Claims

1. A system for manipulating human gut microbiota *in-situ,* comprising a swallowable light emitting vehicle configured to move through the GI tract of a human subject; a software; a software operated controller and a plurality of protocols for manipulating the gut microbiota, wherein each protocol comprises a predefined set of conditions, and, wherein said set of conditions comprises at least one illumination episode at the wavelength in the range of 400nm to 900nm.

2. The system of claim 1, wherein said set of conditions comprises at least one illumination episode at the wavelength in the range of 420nm to 600nm.

3. The system of claim 1, wherein said set of conditions comprises at least one illumination episode at the wavelength in the range of 600nm to 750nm.

4. The system of claim 1, wherein said set of conditions comprises at least one illumination episode at the wavelength in the range of 750 nm to 900nm.

5. The system of any one of claims 1 to 4, wherein the light emitting vehicle comprises at least one light emitting unit, wherein said light emitting unit comprises a plurality of light emitting elements, and, wherein the light emitting unit comprises a plurality of light emitting elements configured to emit a light at the wavelength in the range of 400nm to 900nm.

6. The system of any one of claims 1 to 5, wherein the controller is configured to deliver two or more protocols for manipulating gut microbiota at the desired location.

7. The system of any one of claims 1 to 6, wherein the plurality of protocols are delivered at the same location of the GI tract or at the different locations of the GI tract.

8. The system of any one of claims 1 to 7, wherein the swallowable light emitting vehicle further comprises at least one reservoir configured to carry and release cargo.

9. The system of claim 8, wherein the cargo is a sample of human microbiota.

10. The system of any one of claims 1 to 9, wherein the software operated controller is configured to fulfill at least one of the following functions: regulating the function of the light emitting unit, regulating the motion of the vehicle, identifying the location of the vehicle, instructing the vehicle to reach a desired location of the GI tract; delivering the at least one protocol for manipulating the gut microbiota at the desired location, and, actuating release of the cargo from the at least one reservoir at the desired location.

11. The system of any one of claims 1 to 10, wherein the at least one protocol further comprises at least one of the parameters selected from the group consisting of illumination doses, wavelengths, duration of each illumination, number of illumination episodes, frequency of illumination episodes, exposure to oxygen, humidity, exposure to acoustic wave, pH, exposure to radio frequency, temperature, presence of medication, presence of bacterial species, and presence of bioactive substance.
